# EUROPEAN PATENT APPLICATION

(11) **EP 2 410 601 A1**
(43) Date of publication of application: **25.01.2012**
(21) Application number: 10753526.2
(22) Date of filing: 16.03.2010
(51) Int. Cl.: H01M 10/0568, H01M 10/052

(54) **IONIC LIQUID, ELECTROLYTE, LITHIUM SECONDARY BATTERY COMPRISING SAME, AND PROCESS FOR PRODUCING IONIC LIQUID**

(30) Priority: 18.03.2009 JP 2009065665; 20.11.2009 JP 2009265360
(71) Applicant: The Nippon Synthetic Chemical Industry Co., Ltd., Osaka-shi Osaka 531-0076 (JP)
(72) Inventor: TATSUMI, Ryouta, Osaka-shi Osaka 531-0076 (JP); AOKI, Yasuhiro, Osaka-shi Osaka 531-0076 (JP); MAEDA, Seiji, Osaka-shi Osaka 531-0076 (JP); HORI, Mariko, Osaka-shi Osaka 531-0076 (JP); HAYAKAWA, Seiichirou, Osaka-shi Osaka 531-0076 (JP)
(74) Representative: Kuhnen & Wacker
(86) International application number: PCT/JP2010/054462
(87) International publication number: WO 2010/107034

(57) **Abstract**

The present invention provides an ionic liquid useful for a lithium secondary battery, which is excellent in performance as an electrolyte material, such that the liquid has ionic conductance and a wide potential window, and is excellent in safety, as well as an electrolyte using the same. The present invention relates to an ionic liquid including a cyanophosphate-based anion represented by the following general formula (1), and an electrolyte using the same, a lithium secondary battery, and a process for producing the ionic liquid.

[Chem 1] ⁻P(CN)ₙX₆₋ₙ (1)

(Wherein X is a halogen atom and n is an integer of 1 to 6.)

## Description

### Technical Field

The present invention relates to a novel ionic liquid. More specifically, it relates to an ionic liquid useful for a lithium secondary battery, which is excellent in performance as an electrolyte material, such as ionic conductance and potential window, and excellent in safety, and a process for producing the same, as well as an electrolyte and a lithium secondary battery using the same.

### Background Art

Recently, in information electronic devices such as laptop personal computers, mobile phones, and PDA, lithium secondary batteries have remarkably come into wide use. For seeking more comfortable portability, miniaturization, thinning, weight saving, and increase in performance of the batteries have rapidly advanced. Moreover, also in electric vehicles which are expected to be next-generation automobiles, application of the lithium secondary batteries has been investigated and thus further miniaturization, weight saving, and increase in performance are required.

On the other hand, safety of current lithium secondary batteries, for example, ignition, accidental explosion, or the like of the information electronic devices, has become a problem. Since the current lithium secondary batteries use flammable and volatile organic solvents such as propylene carbonate and diethyl carbonate as electrolytic solutions, the batteries always have danger of ignition and accidental explosion. The danger increases as the capacity of the batteries increases and the danger has inhibited wide use of the electric vehicles.

Therefore, there has been investigated a lithium secondary battery using a flame retardant and low volatile ionic liquid as an electrolyte material instead of the organic solvents. The ionic liquid herein is a salt containing a cation and an anion and is a compound having a melting point of lower than around room temperature.

As an electrolyte using the ionic liquid, there has been, for example, proposed an electrolyte containing an imidazolium-based cation, a lithium cation, and a fluorine-containing anion (e.g., see Patent Document 1). Also, it has been proposed that a specific ionic liquid containing a nitrogen-containing anion can be applied as a material for an ionic conductor (e.g., see Patent Document 2). Moreover, it has been proposed that a specific ionic liquid containing a dicyanamide anion can be applied as a material for an ionic conductor (e.g., see Patent Document 3).

### Background Art Documents

### Patent Documents

Patent Document 1: JP-A-2004-303642
Patent Document 2: JP-T-2001-527505
Patent Document 3: JP-A-2004-6240

### Summary of the Invention

### Problems that the Invention is to Solve

However, in the technology disclosed in the above Patent Document 1, ⁻N(SO₂CF₃)₂, ⁻N(SO₂C₂F₅)₂, ⁻N(SO₂CF₃)(SO₂C₄F₉), or the like is used as an anion of the ionic liquid, but the electrolyte using the ionic liquid including such an anion has a problem that conductive property at around room temperature is secured but conductive property at low temperature is inferior since the melting point is high. Particularly, conductive property at low temperature (e.g., -30°C) necessary as an electrolyte for an automobile battery cannot be secured.

Furthermore, in the case of the electrolyte using such an ionic liquid, since decomposition reactions through oxidation and/or reduction on the cathode surface and/or anode surface of the battery proceed, even when a cathode material and an anode material each having a wide potential window are used, the wide potential window thereof cannot be effectively utilized. For example, the potential window of an ionic liquid composed of an imidazolium-based cation is generally about 3 to 5V but a potential window of 5V or more is necessary for an ionic liquid to be used in an automobile battery. Particularly, in order to secure the safety of the batteries even in an overcharged state, an ionic liquid having a wide potential window even at 0.1V becomes necessary.

Moreover, in the technology disclosed in the above Patent Document 2, ⁻N(SO₂F)₂ or the like is used as an anion of the ionic liquid, but the ionic liquid including such an anion has a large calorific value when decomposed and thus is more dangerous than an organic solvent. For example, temperature at inside of a battery reaches several hundred degrees on occasion resulting from short circuit or the like, and the battery may be exploded or ignited by such thermal runway. When an ionic liquid having a large calorific value is used as an electrolyte material, such thermal runway is to be accelerated.

In the technology disclosed in the above Patent Document 3, a dicyanamide anion or the like is used as an anion of the ionic liquid, but the ionic liquid including such an anion decomposes at a relatively low temperature, so that the ionic liquid also accelerates the thermal runway.

Accordingly, in the present invention, under such backgrounds, an object is to provide an ionic liquid useful for a lithium secondary battery, which is excellent in performance as an electrolyte material, such as ionic conductance and potential window, and excellent in safety, and an electrolyte using the same, as well as a lithium secondary battery.

### Means for Solving the Problems

As a result of extensive studies in consideration of such circumstances, the present inventors have found that an ionic liquid including a novel phosphate-based anion having a cyano group, or a cyano group and a halogen is excellent in performance such as a wide potential window and excellent in safety when used as an electrolyte material, and thus have accomplished the present invention.

That is, the present invention includes the following embodiments.
[1] An ionic liquid comprising a cyanophosphate-based anion represented by the following general formula (1:

[Chem 1] ⁻P(CN)ₙX₆₋ₙ (1)

(wherein X is a halogen atom and n is an integer of 1 to 6).
[2] The ionic liquid of [1], wherein n in the general formula (1) is 3 to 5.
[3] The ionic liquid of [1] or [2], wherein X in the general formula (1) is a fluorine atom.
[4] The ionic liquid of any one of [1] to [3], which comprises a nitrogen-containing organic cation.
[5] The ionic liquid of [4], wherein the nitrogen-containing organic cation is an imidazolium-based cation.
[6] The ionic liquid of any one of [1] to [5], which is produced by cyanidation of a halogenated phosphate-based anion obtained by reacting a halide of an organic or inorganic cation with a phosphorus halide.
[7] The ionic liquid of [6] wherein X in the general formula (1) is a fluorine atom, which is produced, after the cyanidation of the halogenated phosphate-based anion, by further fluorination of the cyano compound.
[8] The ionic liquid of any one of [1] to [7], which has an electrode protective film-forming function to at least one of cathode material surface and anode material surface, when used as an electrolyte material of a lithium secondary battery.
[9] The ionic liquid of any one of [1] to [8], wherein the ionic conductance at -30°C is 0.0001 mS/cm or more.
[10] An electrolyte comprising the ionic liquid (A) of any one of [1] to [9] and an electrolyte salt (B).
[11] The electrolyte of [10], which is used in a lithium secondary battery.
[12] A lithium secondary battery comprising the electrolyte of [10] or [11], a cathode, and an anode, wherein the electrolyte is placed between the cathode and the anode.
[13] A process for producing the ionic liquid of any one of [1] to [9], comprising a step of reacting a halide of an organic or inorganic cation with a phosphorus halide to obtain a halogenated phosphate-based anion, and a step of cyanidation of the halogen of the halogenated phosphate-based anion, in this order.
[14] The process for producing the ionic liquid of [13] wherein X in the general formula (1) is a fluorine atom, which comprises a step of fluorination of the cyano compound after the step of cyanidation of the halogen of the halogenated phosphate-based anion.

### Effects of the Invention

The ionic liquid of the present invention is useful as an electrolyte material, and the electrolyte obtained therefrom is excellent in performance as an electrolyte material, such that the electrolyte has a wide potential window, and thus a lithium secondary battery excellent in safety can be obtained.

### Brief Description of the Drawings

[FIG. 1] Fig. 1 is an IR chart of the ion liquid (A-1) obtained in Example 1.
[FIG. 2] Fig. 2 is a ³¹P-NMR of the ion liquid (A-1) obtained in Example 1.
[FIG. 3] Fig. 3 is an IR chart of the ion liquid (A-2) obtained in Example 2.
[FIG. 4] Fig. 4 is a ³¹P-NMR of the ion liquid (A-2) obtained in Example 2.
[FIG. 5] Fig. 5 is an IR chart of the ion liquid (A-3) obtained in Example 3.
[FIG. 6] Fig. 6 is a ³¹P-NMR of the ion liquid (A-3) obtained in Example 3.
[FIG. 7] Fig. 7 is an IR chart of the ion liquid (A-4) obtained in Example 4.
[FIG. 8] Fig. 8 is a ³¹P-NMR of the ion liquid (A-4) obtained in Example 4.

### Modes for Carrying Out the Invention

The following will explain the present invention in detail.
Incidentally, the ionic liquid in the present invention indicates an ionic substance containing a cation and an anion, which maintains a liquid at a certain temperature at around room temperature. In the present invention, the room temperature usually means a temperature of 25°C.

The ionic liquid (A) of the present invention is an ionic liquid including a cyanophosphate-based anion represented by the following general formula (1).

[Chem 2] ⁻P(CN)ₙX₆₋ₙ (1)

(Wherein X is a halogen atom and n is an integer of 1 to 6.)

X is a halogen atom, preferably a fluorine atom, a chlorine atom, or a bromine atom from the viewpoint of lowering viscosity, more preferably a fluorine atom or a chlorine atom from the viewpoint of the potential window, and particularly preferably a fluorine atom from the viewpoint of chemical stability such as water resistance and heat resistance, solubility of an electrolyte salt, and the like. Two or more kinds of these halogen atoms may be used in combination. n is an integer of 1 to 6, preferably an integer of 2 to 5 from the viewpoint of the melting point, more preferably an integer of 2 to 4 from the viewpoint of ionic conductance, and particularly preferably 3 from the viewpoint of chemical stability.

A main effect of the ionic liquid of the present invention is to have low viscosity and high ionic conductance. Here, chemical consideration is noted in the case where X is a fluorine atom and n is 3 as an example: first, lowering of viscosity is achieved by the constitution that the anion has a fluorine atom and further lowering of viscosity is achieved by the constitution that the anion has an unsymmetrical chemical structure. It is predicted that geometrical isomers (Facial and Meridional) are present as the anion and it is surmised that the presence thereof in a mixed state contributes further lowering of viscosity.

Moreover, a remarkable effect of the ionic liquid of the present invention is to have a function that a minute amount of decomposition products of the ionic liquid cover the surface(s) of the cathode material and/or the anode material to exhibit an electrode protective film-forming function and stabilize the potential window of the battery, when used as an electrolyte material for a lithium secondary battery.

In general, when an ionic liquid is used as an electrolytic solution, the anion is decomposed at the cathode side to lower the oxidation potential of the battery and the cation is decomposed at the anode side to lower the reduction potential of the battery. In both cases, the potential window becomes unstable and charge-discharge properties decrease.

On the other hand, in the case where an organic solvent is used as an electrolytic solution, electrode protective film-forming agents such as vinylene carbonate are generally added. These additives are known to form a stable SEI (Solid Electrolyte Interface) on the electrode material surfaces through decomposition during the use of the battery. Such SEI protects the electrodes and also oxidation and reduction of the electrolytic solution are suppressed, so that a wide potential window which the positive and negative electrodes primarily have can be fully utilized.

The ionic liquid of the present invention has effects similar to the aforementioned electrode protective film-forming agent. Namely, a part of the ionic liquid at the cathode side is decomposed to protect the cathode material surface and also suppress further decomposition of the ionic liquid, and/or a part of the ionic liquid at the anode side is decomposed to protect the anode material surface and also suppress further decomposition of the ionic liquid. As a result thereof, the oxidation and reduction potential of the battery is stabilized, and a wide potential window which the positive and negative electrodes primarily have can be fully utilized.

Although it is not clear by what mechanism the ionic liquid of the present invention forms the SEI, it is presumed that a stable electrode protective film-forming agent is formed by reacting the cyano group and/or halogen of the anion with the electrode surface. Incidentally, the aforementioned electrode protective film-forming agent such as vinylene carbonate generally stabilizes the reduction potential by forming the protective film on the anode, but the ionic liquid of the present invention can form a protective film also on the cathode and can also stabilize the oxidation potential.

The cation of the ionic liquid (A) of the present invention is sufficiently an organic or inorganic cation and is preferably an organic cation, particularly preferably a nitrogen-containing organic cation from the viewpoint of low melting point.

Examples of the nitrogen-containing organic cation include imidazolium-based cations, pyrrolidinium-based cations, piperidinium-based cations, aliphatic quaternary ammonium-based cations, and the like.

Examples of the imidazolium-based cations include disubstituted imidazolium-based cations such as 1,3-dimethylimidazolium, 1-ethyl-3-methylimidazolium, 1-methyl-3-propylimidazolium, 1-butyl-3-methylimidazolium, 1-methyl-3-pentylimidazolium, 1-hexyl-3-methylimidazolium, 1-heptyl-3-methylimidazolium, 1-methyl-3-octylimidazolium, 1-decyl-3-methylimidazolium, 1-dodecyl-3-methylimidazolium, 1-ethyl-3-propylimidazolium, 1-butyl-3-ethylimidazolium, 1-methoxyethyl-3-methylimidazolium, 1-cyanomethyl-3-methylimidazolium, 1-cyanoethyl-3-methylimidazolium, 1-(3-cyanopropyl)-3-methylimidazolium, 1-methyl-3-trifluoromethylimidazolium, 1-methyl-3-perfluoroethylimidazolium, 1-methyl-3-perfluoropropylimidazolium, 1-methyl-3-perfluorobutylimidazolium, 1-ethyl-3-trifluoromethylimidazolium, 1-ethyl-3-perfluoroethylimidazolium, 1-ethyl-3-perfluoropropylimidazolium, 1-ethyl-3-perfluorobutylimidazolium, 1-propyl-3-trifluoromethylimidazolium, 1-perfluoroethyl-3-propylimidazolium, 1-perfluoropropyl-3-propylimidazolium, 1-perfluorobutyl-3-propylimidazolium, 1-butyl-3-trifluoromethylimidazolium, 1-butyl-3-perfluoroethylimidazolium, 1-butyl-3-perfluoropropylimidazolium, 1-butyl-3-perfluorobutylimidazolium, 1,3-bis(trifluoromethyl)imidazolium, 1-perfluoroethyl-3-trifluoromethylimidazolium, 1-perfluoropropyl-3-trifluoromethylimidazolium, 1-perfluorobutyl-3-trifluoromethylimidazolium, 1,3-bis(perfluoroethyl)imidazolium, 1-perfluoroethyl-3-perfluoropropylimidazolium, 1-perfluorobutyl-3-perfluoroethylimidazolium, 1,3-bis(perfluoropropyl)imidazolium, 1-perfluorobutyl-3-perfluoropropylimidazolium, and 1,3-bis(perfluorobutyl)imidazolium; trisubstituted imidazolium-based cations such as 1,2,3-trimethylimidazolium, 1,3,5-trimethylimidazolium, 3-ethyl-1,2-dimethylimidazolium, 3-ethyl-1,3-dimethylimidazolium, 3-ethyl-1,5-dimethylimidazolium, 1,2-dimethyl-3-propylimidazolium, 1,3-dimethyl-2-propylimidazolium, 1,5-dimethyl-3-propylimidazolium, 1-butyl-2,3-dimethylimidazolium, 2-butyl-1,3-dimethylimidazolium, 3-butyl-1,5-dimethylimidazolium, 1,2-dimethyl-3-hexylimidazolium, 1,2-dimethyl-3-octylimidazolium, 1-ethyl-3,4-dimethylimidazolium, 1-isopropyl-2,3-dimethylimidazolium, 3-trifluoromethyl-1,2-dimethylimidazolium, 3-perfluoroethyl-1,2-dimethylimidazolium, 3-perfluoropropyl-1,2-dimethylimidazolium, 3-perfluorobutyl-1,2-dimethylimidazolium, 1,3-bis(trifluoromethyl)-2-methylimidazolium, 1-perfluoroethyl-2-methyl-3-trifluoromethylimidazolium, 2-cyano-1,3-dimethylimidazolium, 2-cyano-1-ethyl-4-methylimidazolium, 2-cyano-1-propyl-4-methylimidazolium, and 1-butyl-2-cyano-4-methylimidazolium, and the like.

Examples of the pyrrolidinium-based cations include N,N-dimethylpyrrolidinium, N-ethyl-N-methylpyrrolidinium, N-methyl-N-propylpyrrolidinium, N-butyl-N-methylpyrrolidinium, N-methyl-N-pentylpyrrolidinium, N-hexyl-N-methylpyrrolidinium, N-methyl-N-octylpyrrolidinium, N-decyl-N-methylpyrrolidinium, N-dodecyl-N-methylpyrrolidinium, N-(2-methoxyethyl)-N-methylpyrrolidinium, N-(2-ethoxyethyl)-N-methylpyrrolidinium, N-(2-propoxyethyl)-N-methylpyrrolidinium, N-(2-isopropoxyethyl)-N-methylpyrrolidinium, and the like.

Examples of the piperidinium-based cations include N,N-dimethylpiperidinium, N-ethyl-N-methylpiperidinium ion, N-methyl-N-propylpiperidinium, N-butyl-N-methylpiperidinium, N-methyl-N-pentylpiperidinium, N-hexyl-N-methylpiperidinium, N-methyl-N-octylpiperidinium, N-decyl-N-methylpiperidinium, N-dodecyl-N-methylpiperidinium, N-(2-methoxyethyl)-N-methylpiperidinium, N-(2-methoxyethyl)-N-ethylpiperidinium, N-(2-ethoxyethyl)-N-methylpiperidinium, N-methyl-N-(2-methoxyphenyl)piperidinium, N-methyl-N-(4-methoxyphenyl)piperidinium, N-ethyl-N-(2-methoxyphenyl)piperidinium, N-ethyl-N-(4-methoxyphenyl)piperidinium, and the like.

Examples of the aliphatic quaternary ammonium-based cations include N,N,N,N-tetramethylammonium, N,N,N-trimethylethylammonium, N,N,N-trimethylpropylammonium, N,N,N-trimethylbutylammonium, N,N,N-trimethylpentylammonium, N,N,N-trimethylhexylammonium, N,N,N-trimethylheptylammonium, N,N,N-trimethyloctylammonium, N,N,N-trimethyldecylammonium, N,N,N-trimethyldodecylammonium, N-ethyl-N,N-dimethylpropylammonium, N-ethyl-N,N-dimethylbutylammonium, N-ethyl-N,N-dimethylhexylammonium, 2-methoxy-N,N,N-trimethylethylammonium, 2-ethoxy-N,N,N-trimethylethylammonium, 2-propoxy-N,N,N-trimethylethylammonium, N-(2-methoxyethyl)-N,N-dimethylpropylammonium, N-(2-methoxyethyl)-N,N-dimethylbutylammonium, and the like.

Among the above nitrogen-containing organic cations, particularly imidazolium-based cations are preferred from the view point of the ionic conductance and further, dialkylimidazolium cations such as 1-ethyl-3-methylimidazolium, 1-propyl-3-methylimidazolium, 1-butyl-3-methylimidazolium and trialkylimidazolium cations such as 1-butyl-2,3-dimethylimidazolium are preferred from the viewpoint of the potential window.

Examples of the organic cations other than the nitrogen-containing ones include phosphonium cations, sulfonium cations, oxonium cations, and the like. Examples of the phosphonium cations include tetramethylphosphonium cation, tetraethylphosphonium cation, tetrapropylphosphonium cation, tetrabutylphosphonium cation, tetraoctylphosphonium cation, trimethylethylphosphonium cation, triethylmethylphosphonium cation, hexyltrimethylphosphonium cation, trimethyloctylphosphonium, triethyl(methoxymethyl)phosphonium, triethyl(methoxymethyl)phosphonium, and the like.

Examples of the sulfonium cations include trimethylsulfonium cation, triethylsulfonium cation, tributylsulfonium cation, diethylmethylsulfonium cation, dimethylpropylsulfonium, dimethylhexylsulfonium, and the like.

In the present invention, the viscosity (25°C) of the ionic liquid (A) is, in the case where it is used as an electrolyte material, preferably 200 mPa·s or lower, further preferably 150 mPa·s or lower, and particularly preferably 100 mPa·s or lower. When the viscosity is too high, the ionic conductance tends to lower. A lower limit of the viscosity is usually 1 mPa·s.

Moreover, the melting point of the ionic liquid (A) is, in the case where it is used as an electrolyte material, preferably 0°C or lower, further preferably -20°C or lower, and particularly preferably -30°C or lower. When the melting point is too high, the electrolyte tends to freeze. A lower limit of the melting point is usually -250°C.

Furthermore, the boiling point of the ionic liquid (A) is, in the case where it is used as an electrolyte material, preferably 200°C or higher, further preferably 300°C or higher, and particularly preferably 400°C or higher. When the boiling point is too low, the safety of the battery tends to deteriorate. An upper limit of the boiling point is usually 600°C.

In addition, the flash point of the ionic liquid (A) is, in the case where it is used as an electrolyte material, preferably 100°C or higher, further preferably 200°C or higher, and particularly preferably 300°C or higher. When the flash point is too low, the safety of the battery tends to deteriorate. An upper limit of the flash point is usually 600°C. Incidentally, the flash point is a value measured in accordance with JIS K 2265 (_1,2,3,4)

Moreover, with regard to the ionic liquid (A) of the present invention, the calorific value per g of the ionic liquid from 25 to 350°C is preferably 500 J/g or less when calorimetric analysis is performed in accordance with JIS K 0129 using DSC (Differential Scanning Calorimetry). When the calorific value exceeds 500 J/g, the safety of the battery tends to deteriorate. The calorific value is more preferably 450 J/g or less, particularly preferably 420 J/g or less from the viewpoint of safety. A lower limit of the calorific value is usually 1 J/g.

The following will explain the process for producing the ionic liquid (A) of the present invention including a cyanophosphate-based anion.

As the process for producing the ionic liquid (A) of the present invention including a cyanophosphate-based anion, there may be, for example, mentioned a process including a reaction of a halide of an organic or inorganic cation, particularly a halide of a nitrogen-containing organic cation such as an imidazolium cation, with a phosphorus halide, and subsequent cyanidation of the resultant halogenated phosphate-based anion.

The following will explain in detail the process for producing the ionic liquid including a cyanophosphate-based anion using a halide of an alkylimidazolium and phosphorus pentachloride as starting materials as one preferable embodiment of the present invention. However, the present invention is not limited thereto and the ionic liquid can be produced in accordance to the following explanation even when the starting materials are different.

The halide of the alkylimidazolium to be a cation source can be obtained by reacting imidazole with an alkyl halide in equivalent amounts to form a quaternary ammonium salt. The halogen is preferably a chlorine atom or a bromine atom.

Then, the halide of the alkylimidazolium is stirred with an equivalent amount of phosphorus pentachloride in a reaction solvent and they are reacted usually at room temperature to 100°C, preferably at room temperature to 50°C usually for several minutes to several hours, preferably for 10 minutes to 1 hour to obtain an intermediate containing an alkylimidazolium cation and a halogenated phosphate-based anion. The reaction is preferably carried out under an inert gas atmosphere, particularly under a dry atmosphere. As the reaction solvent, polar solvents such as acetone, methylethylketone, acetonitrile, dichloromethane, dichloroethylene, chloroform, tetrahydrofuran (THF), dimethylformamide (DMF), dimethylsulfoxide (DMSO) are preferred, and particularly, dehydrated dichloromethane and dehydrated acetonitrile are preferred.

Finally, a cyano compound, for example, 1 to 10 equivalents of a cyano compound per mol of the intermediate containing the alkylimidazolium cation and the halogenated phosphate-based anion, is added to the reaction system, followed by stirring, and the reaction is carried out usually at -30°C to 100°C, preferably at 0°C to 50°C, particularly preferably at 10 to 30°C, usually for several minutes to several tens hours, preferably for 10 minutes to 50 hours, particularly preferably for 1 to 24 hours, whereby an objective ionic liquid (A) including a cyanophosphate anion can be obtained. The reaction is preferably carried out under an inert gas atmosphere, particularly under a dry atmosphere. Examples of the cyano compound include hydrogen cyanide, trimethylsilyl cyanide, potassium cyanide, sodium cyanide, silver cyanide, copper cyanide, and the like. Of these, silver cyanide and copper cyanide are preferred.

In order to remove formed metal halides and impurities, the resultant ionic liquid (A) is preferably purified. Examples of the purification technique include techniques such as filtration, extraction, washing, column chromatograph, and reprecipitation. Of these, column chromatograph is preferred from the viewpoint of improving electrochemical properties of the ionic liquid. As the column, alumina, silica gel, diatomaceous earth, active carbon, and the like may be mentioned. Particularly, from the viewpoint of efficient removability of impurity ions, alumina is preferred.

Thus, using a halide of an alkylimidazolium and phosphorus pentachloride as starting materials, an ionic liquid including an alkylimidazolium cation and a cyano(chloro)phosphate anion is obtained. n in ⁻P(CN)ₙX₆₋ₙ of the general formula (1) can be controlled by the blending amount of the cyano compound, the reaction time, the purification conditions, and the like.

In the case of producing an ionic liquid including a cyano(bromo)phosphate anion, the liquid can be produced in a similar manner using phosphorus pentabromide as a starting material. Moreover, by using phosphorus pentachloride and phosphorus pentabromide in combination as starting materials, an ionic liquid including a cyanochlorobromophosphate anion can be also produced.

Moreover, in the case of producing an ionic liquid including a cyano(fluoro)phosphate anion, the liquid can be produced by further fluorinating the ionic liquid including the cyano(chloro)phosphate anion or the cyano(bromo)phosphate anion produced as mentioned above using a fluorinating agent. More specifically, 3 to 10 equivalents of a fluorinating agent is added to 1 equivalent of the ionic liquid including the cyano(chloro)phosphate anion or the cyano(bromo)phosphate anion and they are reacted usually at 0 to 100°C, preferably at 20 to 80°C, particularly preferably at 25 to 60°C usually for several minutes to several tens hours, preferably for 1 to 24 hours, particularly preferably for 3 to 8 hours, whereby an objective ionic liquid including a cyano(fluoro)phosphate anion can be obtained. As the fluorinating agent, HF, LiF, NaF, KF, AgF, LiBF₄, NaBF₄, KBF₄, AgBF₄, and the like may be mentioned. Of these, AgBF₄ and AgF are preferred. An ionic liquid including a cyano(fluorochloro)phosphate anion or a cyano(fluorobromo)phosphate anion can be also produced by controlling the blending amount of the fluorinating agent and the reaction time of fluorination.

In the case of using a metal salt as a fluorinating agent, in order to remove a metal cation formed as a by-product, it is preferred to perform a post-treatment with adding a metal cation-trapping agent to the resultant ionic liquid. As the metal cation-trapping agent, a compound composed of a cation of the obj ective ionic liquid and a halogen anion. For example, in the case of producing an ionic liquid including 1-ethyl-3-methylimidazolium cation and cyanofluorophosphate anion, a halide of 1-methyl-3-methylimidazolium cation is used. The amount of such a metal cation-trapping agent is preferably 0.1 to 1 equivalent, particularly preferably 0.3 to 0.8 equivalent per equivalent of the fluorinating agent used. As conditions for the post-treatment, the metal cation-trapping agent is added to the ionic liquid, they are reacted usually at 0 to 50°C, preferably 10 to 40°C usually for several minutes to several hours, preferably 10 minutes to 1 hour, then the precipitated metal halide is removed by filtration, and the filtrate is washed with water and dried, whereby a highly pure ionic liquid can be obtained.

Thus, the ionic liquid (A) of the present invention is obtained. With regard to such an ionic liquid (A), in the case where it is used as an electrolyte material, the ionic conductance thereof is preferably 1 mS/cm or more, more preferably 2 mS/cm or more, and further preferably 3 mS/cm or more at 25°C. An upper limit of the ionic conductance at 25°C is usually 100 mS/cm.

Furthermore, in the present invention, the ionic conductance at low temperature is important. For example, the ionic conductance of the ionic liquid (A) at - 30°C is preferably 0.0001 mS/cm or more, more preferably 0.001 mS/cm or more, particularly preferably 0.01 mS/cm or more, and further preferably 0.1 mS/cm or more. An upper limit of the ionic conductance at -30°C is usually 10 mS/cm.

The ionic liquid (A) of the present invention is particularly useful as an electrolyte material. An electrolyte suitable for a lithium secondary battery is provided by containing such an ionic liquid (A) and an electrolyte salt (B).

In the case of obtaining an electrolyte using the ionic liquid (A) of the present invention, the ionic liquid (A) may be used singly or two or more thereof may be used in combination. For example, it is mentioned to use two or more of the ionic liquids, in which X is different from each other or n is different from each other in the general formula (1), in combination. Furthermore, in the case of using two or more of the ionic liquids (A) in combination, it is mentioned to use, for example, as the anion, at least two selected from monofluoropentacyanophosphate anion, difluorotetracyanophosphate anion, trifluorotricyanophosphate anion, tetrafluorodicyanophosphate anion, pentafluoromonocyanophosphate anion, and hexacyanophosphate anion in combination from the viewpoint of lowering the viscosity; and it is mentioned to use, for example, as the cation, at least two selected from 1-ethyl-3-methylimidazolium cation, 1-propyl-3-methylimidazolium cation, 1-butyl-3-methylimidazolium cation, and 1-butyl-2,3-dimethylimidazolium cation in combination from the viewpoint of lowering the melting point.

Examples of the electrolyte salt (B) for use in the present invention include a single compound or mixture of LiBF₄, LiBR₄ (R represents a phenyl group or an alkyl group), LiBFₘR₄₋ₘ (R represents an alkyl group that may contain fluorine atom(s), m is an integer of 1 to 3), LiPF₆, LiSbF₆, LiAsF₆, LiClO₄, LiSO₃CF_{3,} LiN(SO₂CF₃)₂, LiN(SO₂F)₂, LiN(SO₂CF₃)(SO₂F), LiN(CN)₂, LiC(SO₂CF₃)₃, LiSO3C₆F₁₃, LiSO₃C₈F₁₇, LiAlCl₄, lithium tetrakis[3,5-bis(trifluoromethyl)phenyl]borate, a lithium salt of the cyanophosphate-based anion represented by the above general formula (1), and the like. Of these, in view of solubility in the ionic liquid, LiBF₄, LiPF₆, LiN(SO₂CF₃)₂, LiN(SO₂F)₂, and a lithium salt of the cyanophosphate-based anion represented by the above general formula (1) are preferably used.

With regard to the content proportion of the ionic liquid (A) and the electrolyte salt (B), the ionic liquid (A)/the electrolyte salt (B) is preferably 99/1 to 50/50 (ratio by weight), further preferably 95/5 to 60/40 (ratio by weight), and particularly preferably 90/10 to 70/30 (ratio by weight). When the content proportion falls out of the above range, the charge-discharge properties of the lithium secondary battery tend to deteriorate.

In the present invention, in addition to the above ionic liquid (A) and electrolyte salt (B), an ionic liquid (A') other than the ionic liquid (A) can be further contained.

Examples of the ionic liquid (A') other than the ionic liquid (A) include ionic liquids containing a chlorine anion, a bromine anion, an iodine anion, BF₄⁻, BF₃CF₃⁻, BF₃C₂F₅⁻, PF₆⁻, NO₃⁻, CF₃CO₂⁻, CF₃SO₃⁻, (CF₃SO₂)₂N⁻, (FSO₂)₂N⁻, (CF₃SO₂)(FSO₂)N⁻, (CN)₂N⁻, (CN)₃C⁻, (CF₃SO₂)₃C⁻, (C₂F₅SO₂)₂N⁻, AlCl₄⁻, Al₂Cl₇⁻, or the like as an anion. Examples of the cation corresponding thereto includes alkylimidazolium-based cations such as 1,3-dimethylimidazolium, 1-ethyl-3-methylimidazolium, 1-butyl-3-methylimidazolium, 1-hexyl-3-methylimidazolium, 1-octyl-3-methylimidazolium, 1-butyl-2,3-dimethylimidazolium, 1,2,3-trimethylimidazolium, and 1,2-dimethyl-3-ethylimidazolium. Other than the imidazolium-based cations, there are mentioned ionic liquids containing a quaternary ammonium-based cation, a pyridinium-based cation, a quaternary phosphonium-based cation, or the like. These ionic liquids (A') can be used singly or two or more thereof can be used in combination.

With regard to the mixing proportion of the ionic liquid (A) to the other ionic liquid (A'), in the case where the total ionic liquid is taken as 100 parts by weight, the ionic liquid (A') is preferably 50 parts by weight or less, further preferably 30 parts by weight or less, and particularly preferably 20 parts by weight or less. When the ionic liquid (A') is too large in amount, the ionic conductance at low temperature tends to lower and also the electrode protective film-forming function decreases, so that the charge-discharge properties tend to deteriorate.

Furthermore, in the present invention, in order to improve the stability of the potential window and improve the charge-discharge properties in the case where a battery is produced using the electrolyte, it is possible to use an electrode protective film-forming agent (C). Examples of the electrode protective film-forming agent (C) include vinylenecarbonate, 1,3-propanesultone, ethylenesulfite, triethylborate, butyl methylsulfonate, and the like. Of these, from the viewpoint of forming stable SEI, vinylene carbonate is particularly preferred.

The content of the electrode protective film-forming agent (C) is preferably 0.1 to 5 parts by weight, further preferably 0.2 to 3 parts by weight, and particularly preferably 0.3 to 1 part by weight based on 100 parts by weight of the ionic liquid (A). When the content is too large, the safety of the battery tends to deteriorate, and when the content is too small, there is a tendency that the stabilizing effect of the potential window of the lithium secondary battery is not obtained.

Moreover, in the present invention, in order to further improve electric conductivity, it is possible to blend a small amount of an organic solvent. Examples of such an organic solvent include carbonate-based solvents (propylene carbonate, ethylene carbonate, butylene carbonate, dimethyl carbonate, diethyl carbonate, etc.), amide-based solvents (N-methylformamide, N-ethylformamide, N,N-dimethylformamide, N-methylacetamide, N-ethylacetamide, N-methylpyrrodilinone, etc.), lactone-based solvents (γ-butyrolactone, γ-valerolactone, δ-valerolactone, 3-methyl-1,3-oxazolidin-2-one, etc.), alcohol-based solvents (ethyleneglycol, propyleneglycol, glycerin, methyl cellosolve, 1,2-butanediol, 1,3-butanediol, 1,4-butanediol, diglycerin, polyoxyalkylene glycol cyclohexanediol, xyleneglycol, etc.), ether-based solvents (methylal, 1,2-dimethoxyethane, 1,2-diethoxyethane, 1-ethoxy-2-methoxyethane, alkoxypolyalkylene ethers, etc.), nitrile-based solvents (benzonitrile, acetonitrile, 3-methoxypropionitrile, etc.), phosphoric acids and phosphate ester solvents (orthophosphoric acid, metaphosphoric acid, pyrophosphoric acid, polyphosphoric acid, phosphorous acid, trimethyl phosphate, etc.), 2-imidazolidinones (1,3-dimethyl-2-imidazolidinone, etc.), pyrrolidones, sulfolane-based solvents (sulfolane, tetramethylenesulfolane, etc.), furan-based solvents (tetrahydrofuran, 2-methyltetrahydrofuran, 2,5-dimethoxytetrahydrofuran, etc.), dioxolan, dioxane, and the like. A single solvent or a mixed solvent of two or more thereof can be used. Of these, the carbonate-based solvents, the ether-based solvents, the furan-based solvents, and the sulfolane-based solvents are preferred in view of the electric conductivity and, in particular, the sulfolane-based solvents are more preferably used in view of the safety of the battery.

The content in the case of using the organic solvent is preferably 30% by volume or less, particularly 5 to 20% by volume in the case where the whole electrolyte is taken as 100% by volume. When the content is too large, the flame retardancy of the ionic liquid tends to decrease.

The electrolyte of the present invention is produced by mixing the ionic liquid (A) and the electrolyte salt (B), and, if necessary, the other ionic liquid (A'), the electrode protective film-forming agent (C), and further the organic solvent above so as to become a homogeneous solution. Mixing is usually performed in a dry room or a dry box.
Thus, an electrolyte containing the ionic liquid (A) of the present invention and the electrolyte salt (B) is obtained.

The following will explain the lithium secondary battery obtained using the electrolyte of the present invention.
In the present invention, the lithium secondary battery is produced by placing the electrolyte of the present invention obtained in the above between a cathode and an anode.

The cathode is preferably a composite cathode. The composite cathode is a cathode, wherein a composition obtained by mixing a conductive additive such as ketjen black and acetylene black, a binding agent such as polyvinylidene fluoride, and, if necessary, an ion-conductive polymer, to a cathode active material, is applied on a conductive metal plate such as an aluminum foil.

As the cathode active material, an inorganic active material, an organic active material, and a composite thereof can be exemplified but the inorganic active material or the composite of the inorganic active material and the organic active material are preferred from the viewpoint of increase in energy density of the battery.

As the inorganic active material, there are mentioned metal oxides such as Li_{0.3}MnO₂, Li₄Mn₅O₁₂, V_{2O5}, LiFePO₄, and LiMnO₃, and the like as 3V systems and LiCoO₂, LiMn₂O₄, LiNiO₂, LiNi_{1/3}Mn_{1/3}CO_{1/3}O₂, LiNi_{1/2}Mn_{1/2}O₂, LiNi_{0.8}CO_{0.2}O₂, LiMnPO₄, LiMnO₃ and the like as 4V systems, metal sulfides such as TiS₂, MoS₂, and FeS, and composite oxides of these compounds and lithium. As the organic active material, a conductive polymer such as polyacetylene, polyaniline, polypyrrole, polythiophene, or polyparaphenylene, an organic disulfide compound, carbon disulfide, active sulfur, or the like is used.

Examples of the anode include metal-based anodes wherein an anode active material is directly applied on a collector, anodes wherein an active material such as a conductive polymer, a carbon material, or an oxide is applied on an alloy-based collector with a binding material such as polyvinylidene fluoride, and the like.

Examples of the anode active material include metal lithium, metal silicon, alloys of metals such as aluminum, lead, tin, silicon, and magnesium with lithium, metal oxides such as SnO₂ and TiO₂, conductive polymers composed of polypyridine, polyacetylene, polythiophene, or a derivative thereof, which are capable of cation doping, carbon materials capable of occluding lithium, and the like. Of these, particularly, in the case of using the ionic liquid (A) of the present invention, metal lithium and metal silicon having high energy density are preferred.

In the present invention, in the case of using such metal lithium, the thickness of the metal lithium is preferably 1 to 100 µm, further preferably 3 to 50 µm, and particularly 5 to 20 µm. As the metal lithium, it is economically preferred to use a thin lithium foil.

The lithium secondary battery of the present invention is produced by placing the electrolyte between the above cathode and anode, but a separator is preferably used from the viewpoint of prevention of short circuit. Specifically, the lithium secondary battery is obtained by impregnating the separator with the electrolyte and placing the resultant separator between the cathode and the anode.

As the separator, there is used one exhibiting low resistance to ion migration of the lithium ion. Examples thereof include micro porous films composed of one or more materials selected from polypropylene, polyethylene, polyester, polytetrafluoroethylene, polyvinyl alcohol, and saponification products of ethylenevinyl acetate copolymers, and organic or inorganic nonwoven fabrics or woven fabrics. Of these, in view of prevention of short circuit and economical efficiency, a micro porous film composed of polypropylene or polyethylene and a glass nonwoven fabric are preferred.

The shape of the lithium secondary battery of the present invention is not particularly limited, but battery cells having various shapes, such as coins, sheets, and cylinders are mentioned.

### Examples

The following will further specifically explain the present invention with reference to Examples but the present invention is not limited to the following Examples unless the present invention exceeds the gist.
Incidentally, "part(s)" and "%" in Examples means those on the basis of weight unless otherwise stated.
The measurement conditions for each property are as follows.

### (Melting Point)

Using "D2920 Model DSC" manufactured by TA Instruments, an ionic liquid was charged into a pan for only liquid and then was cooled to -150°C under a nitrogen atmosphere. Measurement was performed by elevating the temperature from -150°C to 300°C at a rate of 10°C/minute. Furthermore, with regard to an ionic liquid having a melting point of lower than -30°C by this technique, the liquid was allowed to stand at -30°C for 24 hours and it was confirmed that the liquid was not solidified.

### (Boiling Point)

Measurement was performed by elevating the temperature of an ionic liquid from 25°C to 300°C at a rate of 10°C/minute.

### (Flash Point)

Measurement was performed in accordance with JIS K 2265 (_1, 2, 3, 4).

### (Calorific Value)

Using "DSC 7" manufactured by Perkin Elmer, measurement was performed in accordance with JIS K 0129. After an ionic liquid was charged into a tightly closed pressure pan under an argon atmosphere, the temperature was elevated from 50°C to 350°C at a rate of 5°C/minutes under a nitrogen atmosphere to measure a total calorific value. The measured value was divided by the weight of the ionic liquid to thereby obtain a calorific value per g of the ionic liquid.

### (Ionic Conductivity)

Using CG-511B type cell manufactured by DKK-TOA Corporation as a cell for measurement, after the cell was immersed in an ionic liquid for 5 hours, measurement was performed by an AC impedance method using an electrochemical measurement system "Solartron 1280Z" (manufactured by British Solartron). The measurement was performed at an AC amplitude of 5 mV and a frequency range of 20 k to 0.1 Hz.

### (Potential Window)

A V-4C cell for voltammetry manufactured by BAS Inc. was used as a cell for measurement and electrodes manufactured by BAS Inc. were used as electrodes. Glassy carbon (diameter: 1 mm) was used as a working electrode, platinum was used as a counter electrode, and lithium was used as a reference electrode. Measurement was performed at a potential sweeping rate of 5 mV/sec at a temperature of 25°C. An electrochemical measurement system "Solartron 1280Z" (manufactured by British Solartron) was used as a measurement device. The limiting current density was set to ±1 mA/cm², potential when the density reached +1 mA/cm² was taken as oxidation potential, potential when the density reached -1 mA/cm² was taken as reduction potential, and a difference between the oxidation potential and the reduction potential was taken as the potential window (V).

### (Charge-Discharge Properties)

Using a charge-discharge measurement device manufactured by Keisokuki Center, charging was performed to 4.2 V at a current density of 0.2 mA/cm² and, after a halt for 10 minutes, discharging was performed to 3 V at a current density of 0.2 mA/cm². This charging and discharging were repeated at 25°C and a retention ratio (%) of charging and discharging capacity at 100th cycle was measured.

### Example 1

### [Production of Ionic Liquid (A-1)]

Under an argon stream, 36.4 g of phosphorus pentachloride, 158 mL of dehydrated acetonitrile, and 30.6 g of 1-butyl-3-methylimidazolium chloride were placed in a 300 mL Erlenmeyer flask, followed by stirring for 1 hour. The resultant solution was added into a 1L three-neck round-bottom flask containing 72.3 g of silver cyanide and 142 mL of dehydrated acetonitrile. After stirring for 1 day, the solution was filtrated and the filtrate was concentrated. Then, 100 mL of dichloromethane was added to the concentrate and the whole was washed with 50 mL of water three times. Thereafter, the dichloromethane layer was separated and concentrated to thereby obtain 41.5 g of an ionic liquid (A-1).

The ionic liquid (A-1) was identified by mass spectrometry, IR spectra, and NMR and was confirmed to be objective 1-butyl-3-methylimidazolium trichlorotricyanophosphate.

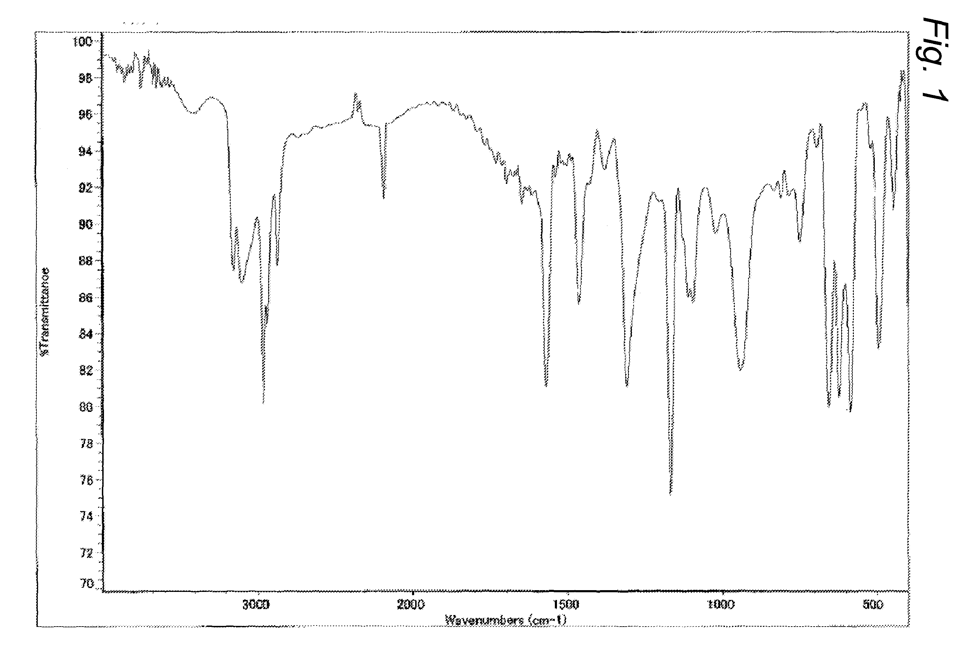

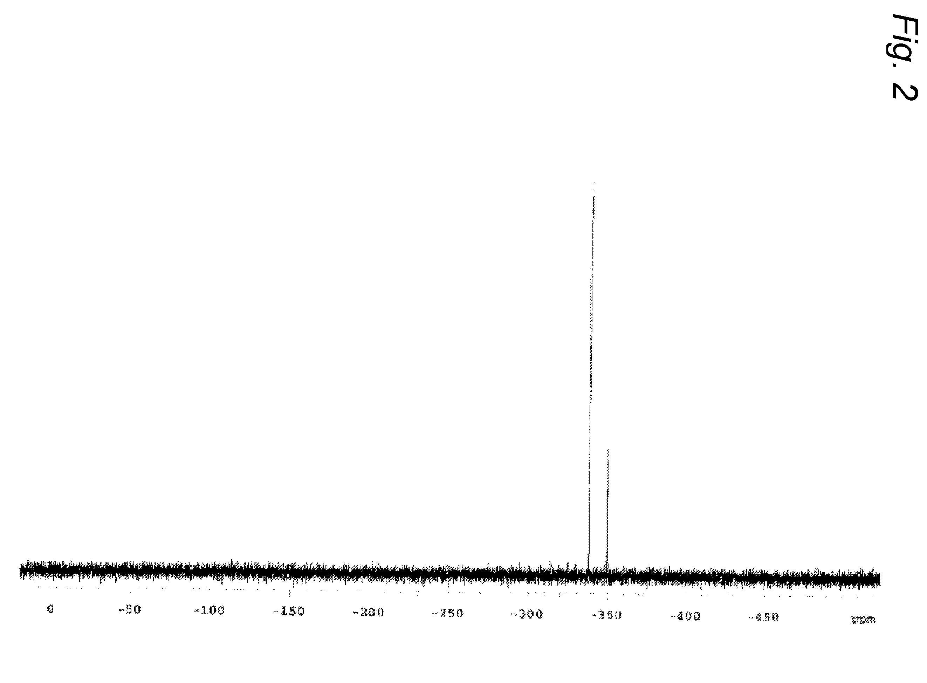

With regard to analyzers, mass spectrometry was measured using "JMS-T100LP AccuTOF LC-plus" manufactured by JOEL Ltd., the IR spectra were measured using "Avatar 360" manufactured by Nicolet, and NMR was measured using "Unity-300" manufactured by Varian (solvent: deuterated dimethyl sulfoxide). Main assignments on charts are shown in the following. Incidentally, the IR spectrum is shown in Fig. 1 and the ³¹P-NMR chart is shown in Fig. 2.

- MS: m/z=213.87557
- IR: 2186 cm⁻¹ [CN]
- ³¹P-NMR: -338.09 ppm [P]
- ¹H-NMR: 9.09 ppm [s, 1H]
7.73 ppm [d, Hz=1.5 Hz, 1H]
7.65 ppm [d, Hz=1.5 Hz, 1H]
4.13 ppm [t, Hz=7.2 Hz, 2H]
3.82 ppm [s, 3H]
1.74 ppm [quint, Hz=7.2 Hz, 2H]
1.24 ppm [sextet, Hz=7.2 Hz, 2H]
0.88 ppm [t, Hz=7.2, 3H]

Various properties of the ionic liquid (A-1) are as shown in Table 1. It was confirmed that the ionic liquid was excellent in electrochemical properties from the fact that the ionic liquid had high ionic conductance even at low temperature and wide potential window.

### Example 2

### [Production of Ionic Liquid (A-2)]

Under an argon stream, 31.7 g of phosphorus pentachloride, 100 mL of dehydrated acetonitrile, and 30.6 g of 1-butyl-2,3-dimethylimidazolium chloride were placed in a 200 mL Erlenmeyer flask, followed by stirring for 1 hour. The resultant solution was added into a 300 mL three-neck round-bottom flask containing 60.9 g of silver cyanide and 60 mL of dehydrated acetonitrile. After stirring for 1 day, the solution was filtrated and the filtrate was concentrated. Then, 100 mL of dichloromethane was added to the concentrate and the whole was washed with 50 mL of water three times. Thereafter, the dichloromethane layer was separated and concentrated to thereby obtain 32.6 g of an ionic liquid (A-2).
Various properties of the ionic liquid (A-2) are as shown in Table 1. It was confirmed that the ionic liquid was excellent in electrochemical properties from the fact that the ionic liquid had high ionic conductance even at low temperature and wide potential window.

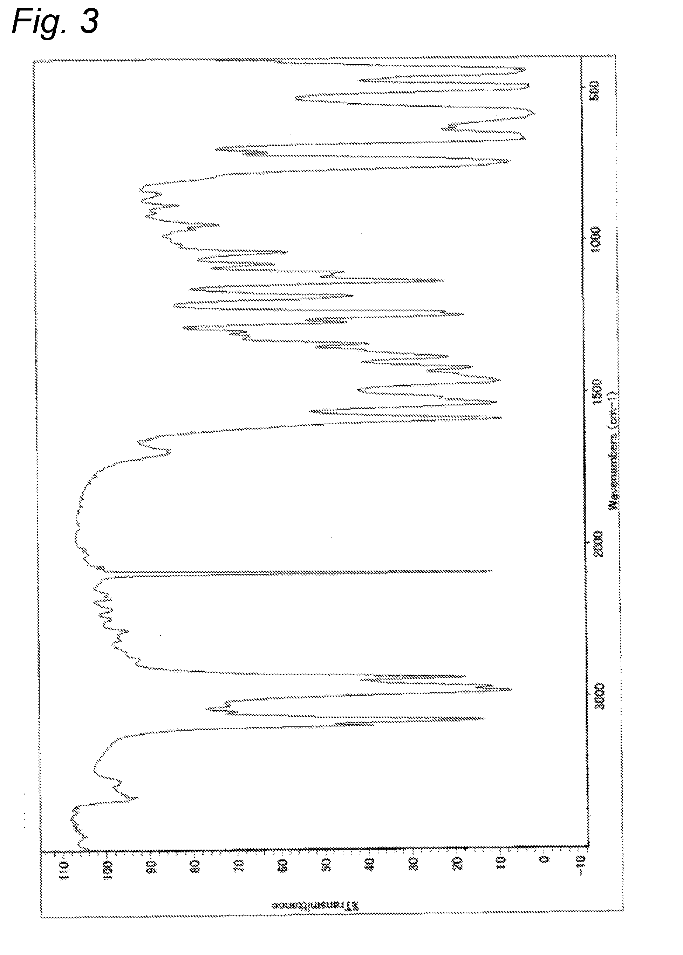

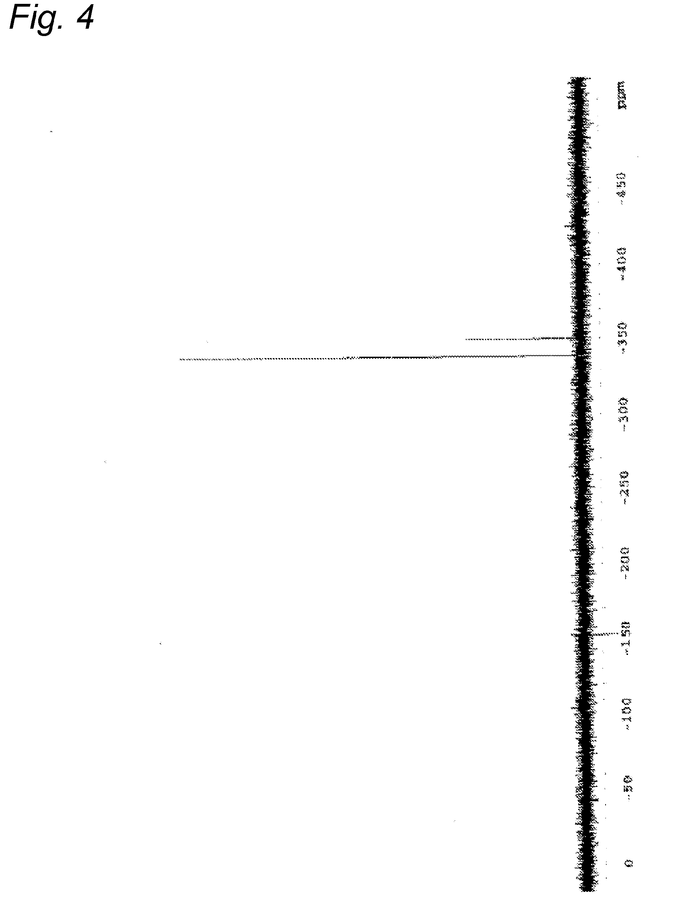

The ionic liquid (A-2) was identified in the same manner as in Example 1 and was confirmed to be objective 1-butyl-2,3-dimethylimidazolium trichlorotricyanophosphate. Main assignments on charts are shown in the following. Incidentally, the IR spectrum is shown in Fig. 3 and the ³¹P-NMR chart is shown in Fig. 4.

- MS: m/z=213.87557
- IR: 2184 cm⁻¹ [CN]
- ³¹P-NMR: -338.09 ppm [P]
- ¹H-NMR: 7.61 ppm [d, Hz=2.4 Hz, 1H]
7.58 ppm [d, Hz=2.1 Hz, 1H]
4.08 ppm [t, Hz=7.3 Hz, 2H]
3.72 ppm [s, 3H]
2.56 ppm [s, 3H]
1.67 ppm [quint, Hz=7.2 Hz, 2H]
1.27 ppm [sextet, Hz=7.2 Hz, 2H]
0.89 ppm [t, Hz=7.3, 3H]

### Example 3

### [Production of Ionic Liquid (A-3)]

Under an argon stream, 22.4 g of 1-butyl-3-methylimidazolium trichlorotricyanophosphate produced in Example 2 and 100 mL of dehydrated acetonitrile were placed in a 200 mL Erlenmeyer flask, and dissolution was achieved. The solution was added into a 500 mL Erlenmeyer flask containing 73.4 g of AgBF₄ and 250 mL of dehydrated acetonitrile. After stirring for 4 days, the solution was filtrated and the filtrate was concentrated. Then, 200 mL of dichloromethane was added to the concentrate and the whole was washed with 100 mL of water four times. Thereafter, the dichloromethane layer was extracted and then dried under vacuum to thereby obtain 10.3 g of an ionic liquid (A-3).
Various properties of the ionic liquid (A-3) are as shown in Table 1. It was confirmed that the ionic liquid was excellent in electrochemical properties from the fact that the ionic liquid had high ionic conductance even at low temperature and wide potential window.

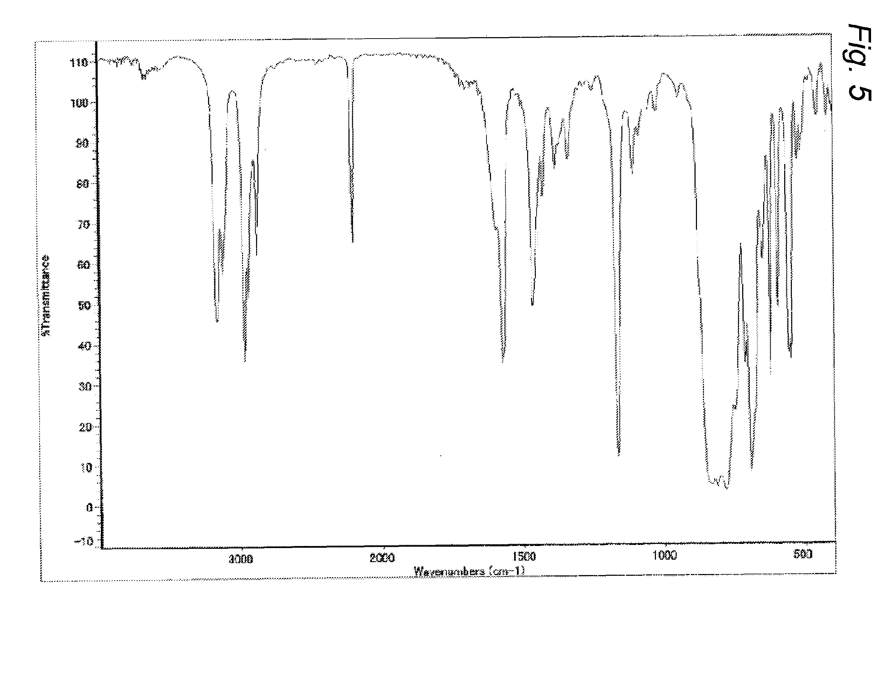

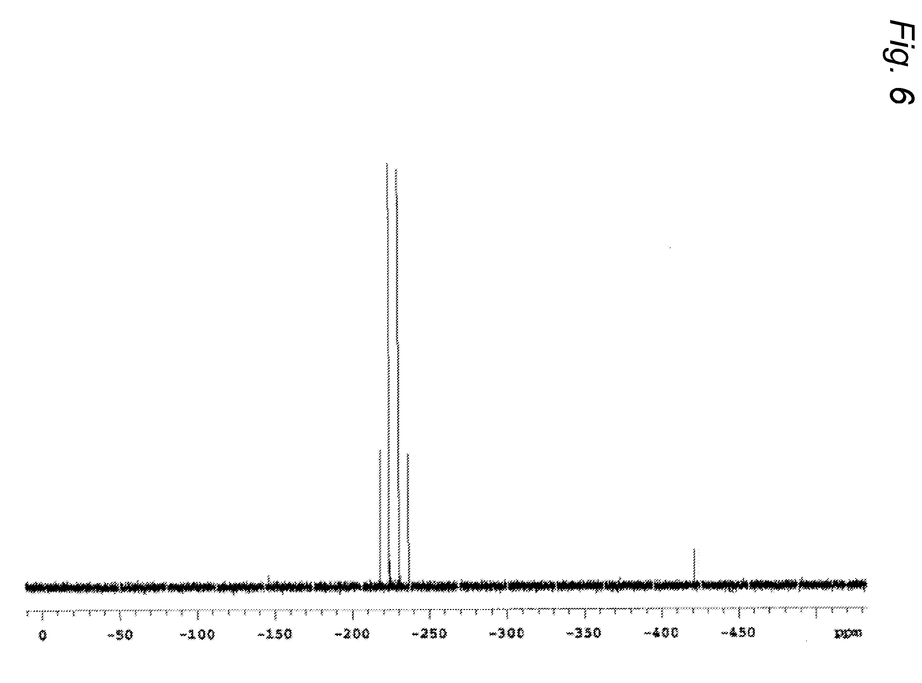

The ionic liquid (A-3) was identified in the same manner as in Example 1 except that deuterated acetonitrile was used as an NMR solvent and was confirmed to be objective 1-butyl-3-methylimidazolium trifluorotricyanophosphate. Main assignments on charts are shown in the following. Incidentally, the IR spectrum is shown in Fig. 5 and the ³¹P-NMR chart is shown in Fig. 6.

- MS: m/z=166.00182
- IR: 2199 cm⁻¹ [CN]
- ³¹P-NMR: -227.09 ppm [q, Hz=739.6 Hz, P]
- ¹H-NMR: 8.42 ppm [s, 1H]
7.35 ppm [d, Hz=1.5 Hz, 1H]
7.32 ppm [d, Hz=1.5 Hz, 1H]
4.09 ppm [t, Hz=7.5 Hz, 2H]
3.79 ppm [s, 3H]
1.76 ppm [quint, Hz=7.5 Hz, 2H]
1.27 ppm [sextet, Hz=7.2 Hz, 2H]
0.89 ppm [t, Hz=7.5 Hz, 3H]

### Example 4

### [Production of Ionic Liquid (A-4)]

Under an argon stream, 41.7 g of phosphorus pentachloride, 140 mL of dehydrated acetonitrile, and 29.5 g of 1-ethyl-3-methylimidazolium chloride were placed in a 300 mL round-bottom flask, followed by stirring for 1 hour. The resultant solution was added into a 500 mL two-neck round-bottom flask containing 81.7 g of silver cyanide and 150 mL of dehydrated acetonitrile. After stirring for 1 day, the solution was filtrated and the filtrate was concentrated. Then, 100 mL of dichloromethane was added to the concentrate and the whole was washed with 50 mL of water three times. Thereafter, the dichloromethane layer was separated and concentrated to thereby obtain 41.4 g of an ionic liquid of 1-ethyl-3-methylimidazolium trichlorotricyanophosphate.
Then, under an argon stream, 38.8 g of 1-ethyl-3-methylimidazolium trichlorotricyanophosphate produced in the above and 70 mL of dehydrated acetonitrile were placed in a 500 mL Erlenmeyer flask, and dissolution was achieved. The solution was added into a 1L Erlenmeyer flask containing 146 g of AgBF₄ as a fluorinating agent and 500 mL of dehydrated acetonitrile. After stirring at 60°C for 5 hours, a solution containing 56 g of 1-ethyl-3-methylimidazolium chloride as a metal cation-trapping agent dissolved in 40 mL of dehydrated acetonitrile was further added thereto, followed by stirring at 25°C for 30 minutes. After the reaction, the reaction solution was filtrated and the filtrate was concentrated. Then, the concentrate was dissolved in 200 mL of dichloromethane and the whole was washed with 100 mL of water four times. The dichloromethane layer was extracted and then dried under vacuum to thereby obtain 16.6 g of an ionic liquid (A-4).
Various properties of the ionic liquid (A-4) are as shown in Table 1. It was confirmed that the ionic liquid was excellent in electrochemical properties from the fact that the ionic liquid had high ionic conductance even at low temperature and wide potential window.

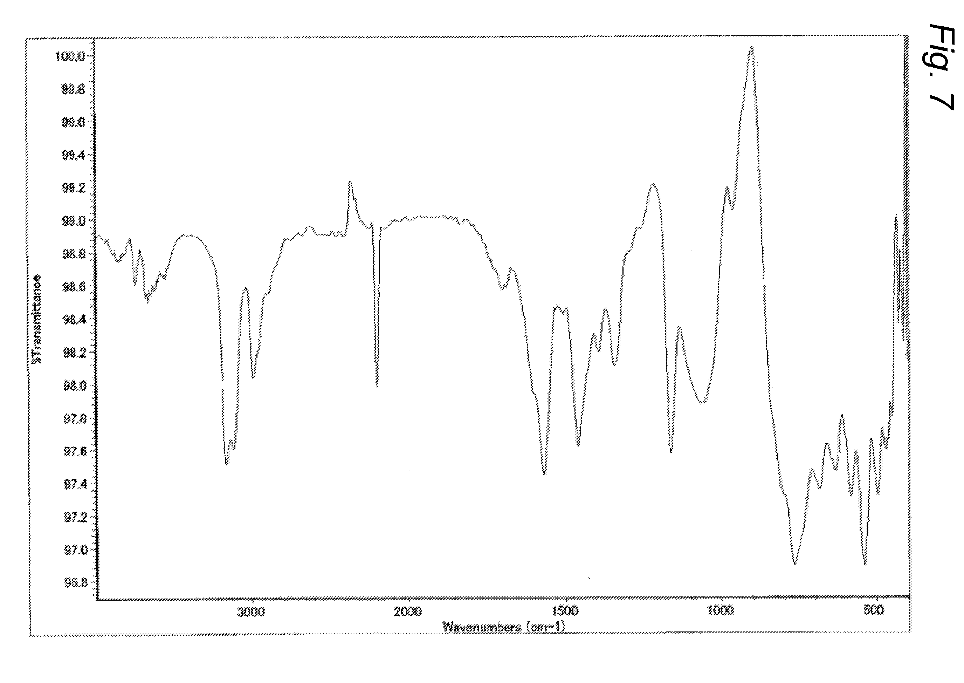

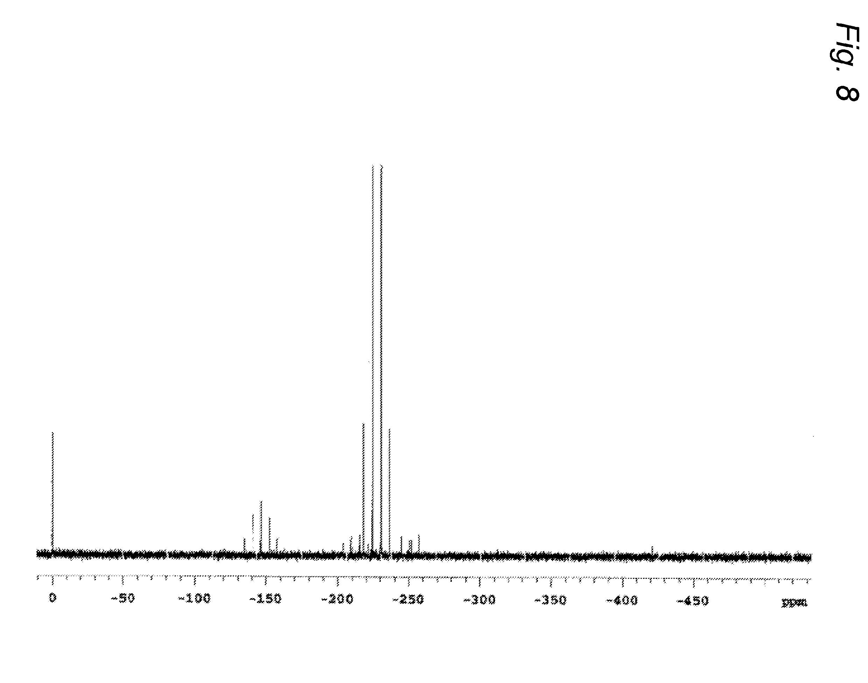

The ionic liquid (A-4) was identified in the same manner as in Example 1 except that deuterated acetonitrile was used as an NMR solvent and was confirmed to be objective 1-ethyl-3-methylimidazolium trifluorotricyanophosphate. Main assignments on charts are shown in the following. Incidentally, the IR spectrum is shown in Fig. 7 and the ³¹P-NMR chart is shown in Fig. 8.

- MS: m/z=165.97917
- IR: 2200 cm⁻¹ [CN]
- ³¹P-NMR: -227.06 ppm [q, Hz=739.6 Hz, P]
- ¹H-NMR: 8.35 ppm [s, 1H]
7.34 ppm [s, 1H]
7.29 ppm [s, 1H]
4.15 ppm [q, Hz=7.2 Hz, 2H]
3.78 ppm [s, 3H]
1.42 ppm [t, Hz=7.2, 3H]

### Comparative Example 1

Evaluation was performed in the same manner as in Example 1 except that the ionic liquid (A-1) was changed to 1-ethyl-3-methylimidazolium bis(trifluoromethanesulfonyl)amide.
The results are as shown in Table 1.

### Comparative Example 2

Evaluation was performed in the same manner as in Example 1 except that the ionic liquid (A-1) was changed to 1-ethyl-3-methylimidazolium bis(fluorosulfonyl)amide.
The results are as shown in Table 1.

### Comparative Example 3

Evaluation was performed in the same manner as in Example 1 using ethylene carbonate (50% by volume)/dimethyl carbonate (50% by volume) that is a common organic electrolytic solution.
The results are as shown in Table 1.

**[Table 1]**

| | | | | | | | |
|---|---|---|---|---|---|---|---|
| <Properties of Ionic Licuid> | | | | | | | |

| | Melting point (°C) | Boiling point (°C) | Flash point (°C) | Calorific value (J/g) | Ionic conductance at 25°C (mS/cm) | Ionic conductance at -30°C (mS/cm) | Potential window (V) |
|---|---|---|---|---|---|---|---|
| Example 1 | <-30 | >400 | >300 | - | 2 | 0.02 | 6 |
| Example 2 | <-30 | >400 | >300 | - | 1 | 0.001 | 6 |
| Example 3 | <-30 | >400 | >300 | 440 | 4 | 0.07 | 6 |
| Example 4 | <-30 | >400 | >300 | 410 | 14 | 1.07 | 5 |
| Comparative Example 1 | -18 | >400 | >300 | - | 9 | impossible to measure owing to freeze | 5 |
| Comparative Example 2 | -19 | >400 | >300 | 630 | 17 | impossible to measure owing to freeze | 5 |
| Comparative Example 3 | - | 150 | 24 | - | - | - | - |

| | | | | | | | |
|---|---|---|---|---|---|---|---|
| Note) "-" in the table indicates that the data was not measured. | | | | | | | |

### Example 5

### [Preparation of Electrolyte]

An electrolyte [I] was obtained by mixing and dissolving 2 g of LiN(SO₂CF₃)₂ as an electrolyte salt into 8 g of the ionic liquid (A-3) produced in Example 3. The electric conductivity of the resultant electrolyte [I] at 25°C was 2 mS/cm.

### [Production of Lithium Secondary Battery]

### (1) Preparation of Cathode

A cathode slurry was obtained by mixing 9.0 g of LiCoO₂ powder, 0.5 g of ketjen black, and 0.5 g of polyvinylidene fluoride, further adding 7.0 g of 1-methyl-2-pyrrolidone, and thoroughly mixing them in a mortar. The resultant cathode slurry was applied on an aluminum foil having a thickness of 20 µm in the air using a wire bar. After dried at 100°C for 15 minutes, the whole was further dried under reduced pressure at 130°C for 1 hour to prepare a composite cathode having a film thickness of 30 µm.

### (2) Assembly of Battery

After a separator (Celgard #2400 manufactured by Celgard, thickness: 20 µm) and the composite cathode were impregnated with the electrolyte [I], the separator and a lithium foil (thickness: 500 µm) as an anode were overlaid on the composite cathode in this order and the resultant one was inserted into a 2032 type coin cell and the cell was sealed to obtain a lithium secondary battery.

### [Evaluation of Lithium Secondary Battery]

As a result of measuring the charge-discharge properties of the resultant lithium secondary battery, the capacity retention ratio was 85% or more and thus the battery had a good charge-discharge capacity retention ratio.

### Example 6

### [Preparation of Electrolyte]

An electrolyte [II] was obtained by mixing and dissolving 2 g of LiN(SO₂CF₃)₂ as an electrolyte salt (B) into 8 g of the ionic liquid (A-4) produced in Example 4. The electric conductivity of the resultant electrolyte [II] at 25°C was 6 mS/cm.

### [Production of Lithium Secondary Battery]

A lithium secondary battery was obtained in the same manner as in Example 6 except that the electrolyte [I] was changed to the electrolyte [II].

### [Evaluation of Lithium Secondary Battery]

As a result of measuring the charge-discharge properties of the resultant lithium secondary battery, the capacity retention ratio was 85% or more and thus the battery had a good charge-discharge capacity retention ratio.

As is clear from the above evaluation results of Examples and Comparative Examples, although the ionic liquids of Comparative Examples had a potential window of about 5 V, all the ionic liquids of Examples 1 to 4 had such a wide potential window as 5 V or more, or 6 V in some cases, and hence are very useful as electrolyte materials of lithium secondary batteries. Moreover, also from the evaluation results of Examples 5 and 6, it is understood that the batteries were very good as lithium secondary batteries. Furthermore, with regard to the ionic conductance, although the ionic liquids of Comparative Examples had good ionic conductance at 25°C but freezed at -30°C, the ionic liquids of Examples 1 to 4 showed good ionic conductance without freezing even at -30°C and thus it is understood that the liquids are useful as materials of electrolytes for automobiles.

While the present invention has been described in detail and with reference to specific embodiments thereof, it will be apparent to one skilled in the art that various changes and modifications can be made therein without departing from the spirit and scope thereof.
The present application is based on Japanese Patent Application No. 2009-065665 filed on March 18, 2009 and Japanese Patent Application No. 2009-265360 filed on November 20, 2009, and the contents are incorporated herein by reference.

### Industrial Applicability

The ionic liquid (A) including a cyanophosphate-based anion of the present invention is excellent in performance as an electrolyte material, such that the liquid has ionic conductance and a wide potential window, and further excellent in safety and thus are very useful as an electrolyte material for lithium secondary batteries. Moreover, the ionic liquid is also useful as an electrolyte material for other secondary batteries, primary batteries, capacitors, condensers, actuators, electrochromic elements, various sensors, dye sensitizing solar batteries, and fuel batteries. Furthermore, the ionic liquid is also useful as a solvent, a heat transfer medium, an ink additive, an antistatic agent, a lubricant, a polymerization initiator, a material for ion-exchange membranes, a material for ion glass, and a gas absorbent.

## Claims

1. An ionic liquid comprising a cyanophosphate-based anion represented by the following general formula (1):
[Chem 1] ⁻P(CN)ₙX₆₋ₙ (1)
(wherein X is a halogen atom and n is an integer of 1 to 6).

2. The ionic liquid according to claim 1, wherein n in the general formula (1) is 3 to 5.

3. The ionic liquid according to claim 1 or 2, wherein X in the general formula (1) is a fluorine atom.

4. The ionic liquid according to any one of claims 1 to 3, which comprises a nitrogen-containing organic cation.

5. The ionic liquid according to claim 4, wherein the nitrogen-containing organic cation is an imidazolium-based cation.

6. The ionic liquid according to any one of claims 1 to 5, which is produced by cyanidation of a halogenated phosphate-based anion obtained by reacting a halide of an organic or inorganic cation with a phosphorus halide.

7. The ionic liquid according to claim 6 wherein X in the general formula (1) is a fluorine atom, which is produced, after the cyanidation of the halogenated phosphate-based anion, by further fluorination of the cyano compound.

8. The ionic liquid according to any one of claims 1 to 7, which has an electrode protective film-forming function to at least one of cathode material surface and anode material surface, when used as an electrolyte material of a lithium secondary battery.

9. The ionic liquid according to any one of claims 1 to 8, wherein the ionic conductance at -30°C is 0.0001 mS/cm or more.

10. An electrolyte comprising the ionic liquid (A) according to any one of claims 1 to 9 and an electrolyte salt (B).

11. The electrolyte according to claim 10, which is used in a lithium secondary battery.

12. A lithium secondary battery comprising the electrolyte according to claim 10 or 11, a cathode, and an anode, wherein the electrolyte is placed between the cathode and the anode.

13. A process for producing the ionic liquid according to any one of claims 1 to 9, comprising a step of reacting a halide of an organic or inorganic cation with a phosphorus halide to obtain a halogenated phosphate-based anion, and a step of cyanidation of the halogen of the halogenated phosphate-based anion, in this order.

14. The process for producing the ionic liquid according to claim 13 wherein X in the general formula (1) is a fluorine atom, which comprises a step of fluorination of the cyano compound after the step of cyanidation of the halogen of the halogenated phosphate-based anion.
